# EUROPEAN PATENT APPLICATION

(11) **EP 2 011 456 A1**
(43) Date of publication of application: **07.01.2009**
(21) Application number: 08251993.5
(22) Date of filing: 09.06.2008
(51) Int. Cl.: A61F 2/44

(54) **Motion restoring intervertebral prosthesis with limited angular displacement**

(30) Priority: 03.07.2007 US 825044
(71) Applicant: SeaSpine, Inc., Vista, CA 92081-7862 (US)
(72) Inventor: Cordaro, Nicholas M., Vista, California 92081 (US); Lauryssen, Carl, Beverly Hills, California 90210 (US); Smith, Colin M., Dana Point, California 92629 (US); Anderson, David G., Morrestown, New Jersey 08057 (US)
(74) Representative: Robson, Aidan John

(57) **Abstract**

A motion restoring intervertebral prosthesis for replacing a failed or partially failed natural disc comprises two components (10,12) defining an imaginary longitudinal axis generally aligned with the spinal axis when installed, the components having vertebral body engaging surfaces on one side and mating articulating surfaces (10c,12c) on the other side, with peripheral rims (10d,12d) encircling the articulating surfaces. The articulating surfaces accommodate rotational motion about the longitudinal axis and limited pivotal motion about the A/P and M/L planes. The rims are arranged to contact and limit the pivotal motion between the components to about +/-6° and preferably +/-4° in both directions in the M/L plane and in the A/P plane in the forward direction. Optionally, the encircling rims allow pivotal action of about 20° to 30° in the A/P plane in the rearward direction.

## Description

### FIELD OF THE INVENTION

The present invention relates to a motion restoring intervertebral prosthesis in the form of an artificial intervertebral disc.

### BACKGROUND OF THE INVENTION

The vertebral bodies forming the spinal column are connected with one another via intervertebral discs which allow the spinal column to move or bend forward and backward, i.e., along the anterior-posterior plane (A/P plane) and sideways along the medial-lateral plane (M/L plane). The column can also pivot at angles intermediate such planes.

A partially or wholly failed natural disc resulting from the imposition of excessive loads or perhaps simply aging can result in considerable back pain for the unlucky individual. The failed disc can be surgically removed and replaced with a rigid solid spacer to maintain the natural spacial separation between the vertebrae; however, such a spacer greatly limits the ability of the vertebrae adjacent the implanted spacer to move relative to each other. Many artificial discs have been proposed in an attempt to restore the natural motion between the vertebral bodies between which a natural disc has been removed. Perhaps the most common type is in the form of a ball and socket. See Hoffmann-Daimler's 1974 article published in Intervertebral Disk Replacement, Vol. 112, No. 4, August 1974. Yuan et al., U.S. Patent No. 5,676,701 ("'701 patent"), discloses a low wear artificial spinal disc having opposing convex and concave contoured surfaces with a full 360 degree circumference. This allows for rotation but no translation. Nishijima et al, U.S. Patent No. 5,899,941, discloses a similar device but where the articulating surface is generally non-conforming to allow rotation and translational movement. This is similar to the intervertebral device disclosed in U.S. Patent No. 6,113,637 by Gill ct al. This artificial spinal disc incorporates a ball and trough type articulation with a substantially flat portion. Both the Nishijima and Gill et al. patents describe devices which rely on point contact, a potential source of excessive wear debris. Also see German Patent No. 2,263,842.

The prior art ball and socket intervertebral devices of which 1 am aware allow for a wide range of motions (ROM) of the order of 20° - 22° between the separated vertebrae or +/- 10° and 11 ° bending in all directions. See the Bryan artificial cervical disc. (Hoang Le et al, Neurosurg Focus 17 (3)) and the Prestige II cervical disc (Porchet et al, Neurosurg Focus 17(3)). Also see the '701 ('''701 patent") in which the inventors state that a natural healthy disc experiences a limit of about 11° motion in the A/P plane, i.e., bending forward/backward and about 3° - 5° in the M/L plane, i.e., bending side to side, and that some skilled artisans considered that artificial discs should have such limitations.

Apparently, the 11° motion in the A/P plane represents the angle deviation from each side of neutral or +/- 11° This is consistent with the '701 patent's reference to 20° in flexion in the design disclosed in U.S. Patent No. 4,759,769 in which the backward angle B (Fig. 8) is limited to approximately 20° ('769 patent at 4:27-29). These permissible A/P angles are consistent with the range of motions discussed in the Neurosurg Focus paper. The 3°-5° motion in the M/L plane alluded to in the '701 patent is inconsistent with the motion of commercial ball and socket intervertebral devices of which 1 am aware, those discussed in the Neurosurg Focus paper and with the teachings in the '701 patent, i.e, that such restricted motion is "not needed." The '071 patent discloses an artificial spinal disc which allows "a considerable angle of flexion/extension generally of about 20° to 30°" (4:38-39)

The large range of motion 20°-22° discussed in the Neurosurg Focus paper and the 20° and advocated in the '701 patent conforms to standard and customary practices for all large joint arthroplasty devices (hips, knees, shoulders). The problem with the outer perimeter or outer rim of an artificial joint contacting is wear debris and implant loosening. This is especially true with hip replacements. If a patient moves his or her leg out past what the artificial hip joint can accommodate, a large moment arm begins to wedge the implant out of position while at the same time generating rim wear from surfaces contacting which were not designed to contact

The problem with transferring the design philosophy of large joint prosthesis to spinal arthroplasty is that the natural disc only moves a small amount, i.e., about 10°-12° total ROM. In addition, natural discs resist excessive motion whereas current articulating type artificial discs do not resist motion above such limits.

Some orthopedic spinal specialists are starting to see a fair amount of induced segmental kyphosis and scoliotic curvatures with both cervical and lumbar disc arthroplasty. See, for example, the October 2006 issue of "J Spinal Disorders and Techniques, pages 466-469.

There is a need for a intervertebral prosthesis which more closely matches the range of motion of a natural disc.

### SUMMARY OF THE INVENTION

A motion restoring intervertebral prosthesis for placement between adjacent vertebrae, between which a natural disc has been partially or completely removed, in accordance with the present invention includes first and second articulating components forming an intervertebral prosthetic disc. Each component has a vertebral body engaging surface on one side for buttressing against a respective vertebral body and an articulating surface on the other side joined with an encircling rim section. One of the articulating surfaces is in the form of an outwardly projecting convex section forming, for example, a partial spherical surface and the other articulating surface is in the form of a mating inwardly extending concave recessed section forming, for example, a partial spherical surface. The rim sections arc arranged to contact each other to limit the angle through which the components can pivot relative to each other in both directions in M/L plane and in a forward direction in the A/P plane to about +/- 6° or less, i.e, +/- 3° - 4°. To inhibit the disc from exiting the space between the separated vertebrae during excessive backwards bending the posterior portions of the rim sections may be chamfered to allow bending in that direction within the range of about 20° to 30°. The prosthesis is basically in the form of a ball and socket, allowing rotation about the spinal axis, with encircling rims which contact each other to limit the bending motion in the M/L and A/P directions. As an option the concave recess can be provided with a nonconforming radius to allow limited translation in the anterior-posterior direction as is explained in some detail in pending PCT Application No. US0616392 assigned to the assignee of this application, the contents of such application are incorporated herein by reference.

### BRIEF DESCRIPTION OF THE DRAWINGS

Fig. 1 is a perspective view of the first and second components of an unassembled intervertebral prosthetic disc in accordance with the present invention;

Fig. 2 is a plan view of the first and second components showing the vertebral body engaging surface;

Fig. 3 is a plan view of the first component of Fig. 1 showing the central section thereof which defines the articulating surface and the encircling rim section;

Fig. 4 is a plan view of the second component showing the central section thereof which defines the articulating surface and the encircling rim section;

Fig. 5 is a M/L side elevational view of the assembled disc of Fig. 1 showing the permissible angle β through which the components can pivot relative to each other in the M/L plane;

Fig. 6 is an A/P side elevational view of the assembled disc of Fig. 1 with the second (lower) component in cross-section;

Figs. 7 and 8 are side elevational views of the disc of Fig. 1 taken along the A/P plane with the second component in cross-section showing the permissible angles ⊖ and ∝, respectively, through which the components can pivot relative to each other in the that plane;

Fig. 9 is a plan view looking at the articulating surface and encircling rim section of a second (lower) component of another embodiment of an intervertebral prosthetic disc showing a sector of a spherical surface in the M/L plane and a modified spherical surface with a sweeping radius in the A/P plane;

Figs. 10 and 11 are side elevational views of an intervertebral disc in which the first (upper) component of Fig. 1 is mated with the second lower component of Fig. 9 taken along the M/L and A/P planes, respectively; and

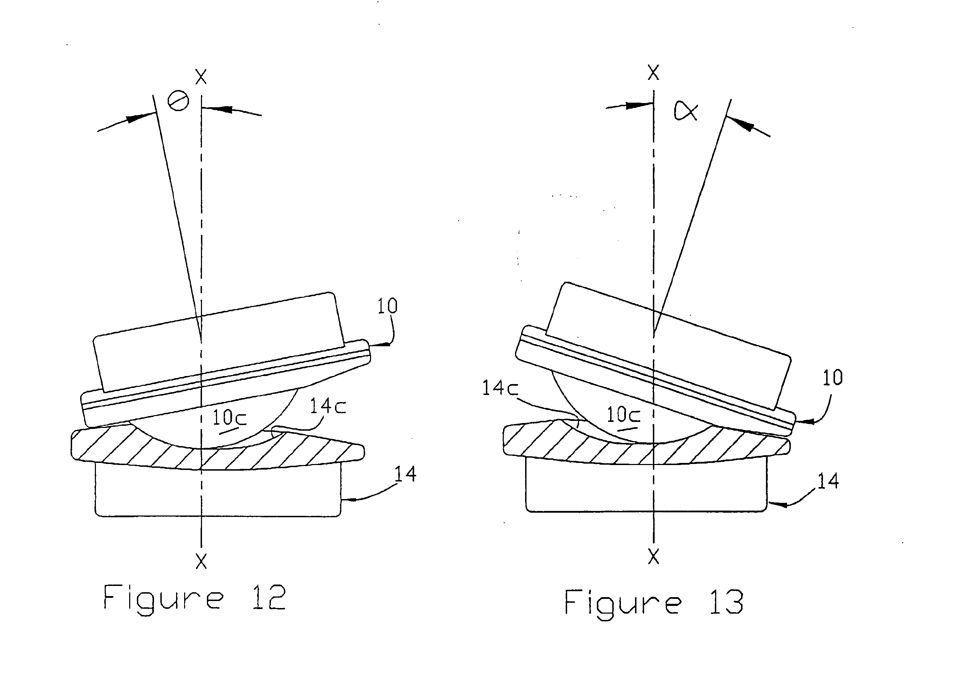

Figs. 12 and 13 are side elevational views of the disc of Figs. 10 and 11 with the lower component in cross-section showing the ability of the first component to pivot and pivot and translate relative to the lower component in the M/L and A/P planes, respectively

### DESCRIPTION OF THE PREFERRED EMBODIMENT

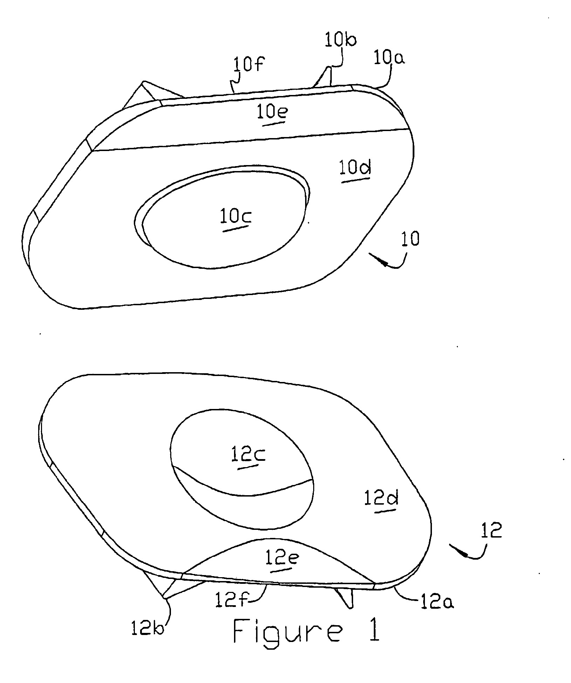

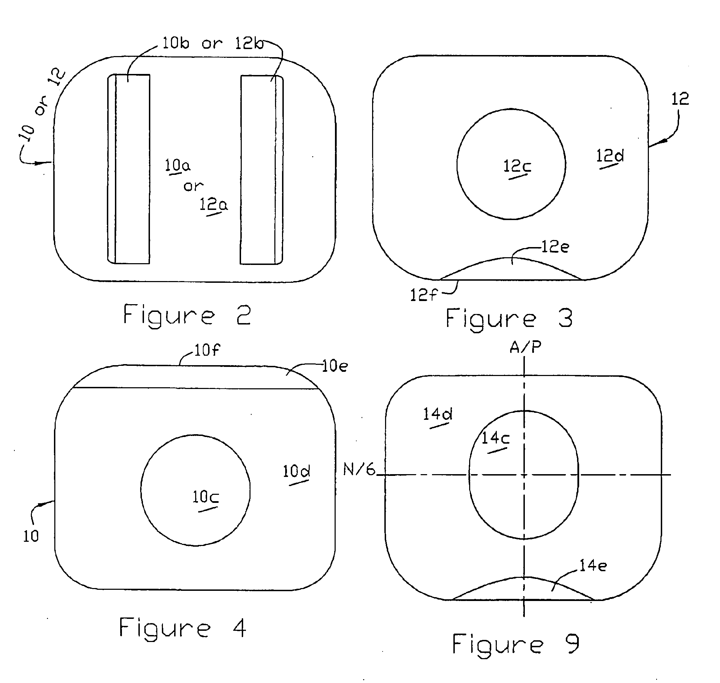

Referring now to Figs. 1-7 an intervertebral prosthesis comprises first and second components 10 and 12, with each of the components having, on one side, vertebral body engaging surfaces 10a and 12a, respectively, (Fig. 2) for buttressing against the exposed faces of the separated vertebrae. The vertebral body engaging surfaces include keels 10b and 12b to aid in anchoring the components to the bone end faces of respective vertebrae.

Component 10 has an articulating surface 10c in the form of a centrally located generally semispherical convex center section joined to or merged with an encircling rim section 10d. The rim section includes a beveled portion 10e on the posterior side 10f (as installed).

Component 12 has an articulating surface 12c in the form of a generally semi-spherical convex or recessed center section functioning as a socket, for mating with the ball section 10c. The articulating socket 12c is joined to or merged with an encircling rim section 12d, which rim section is formed with a beveled portion 12e along the posterior side 12f. The beveled portions 10e and 12e are slanted upwardly and downwardly, respectfully, as shown in the figures. The actual orientation will depend on which component is located on top.

The encircling rims while illustrated as generally rectangular with rounded corners can be any desired shape.

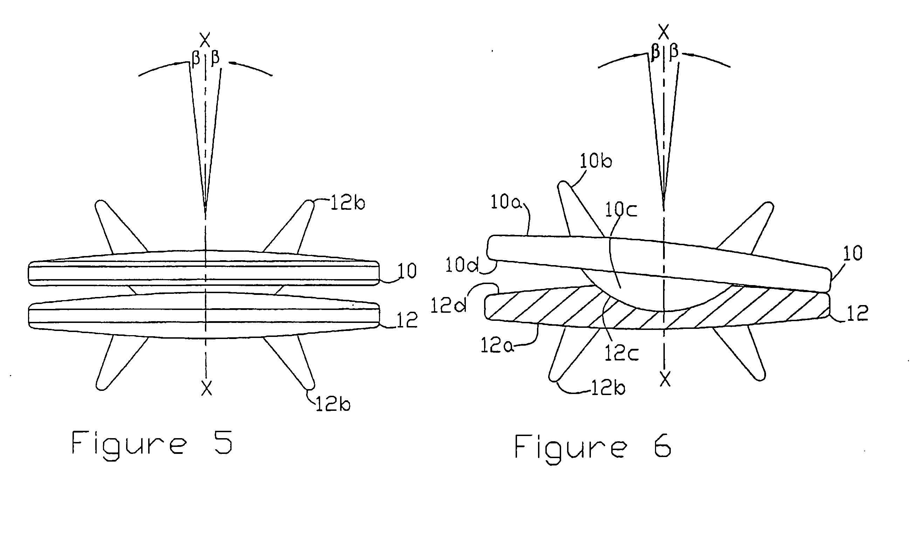

Looking at the assembled disc along the M/L plane in Figs. 5 and 6, the encircling rims contact each other when the components have pivoted relative to each other through an angle β which is within the range of about 12° ROM or +/- 6° and preferably within the range of 8° ROM or +/- 4° (or even +/-3°) from the longitudinal axis x-x (along the spine) in the M/L plane representing a patient bending from side to side. This rim to rim contact thereby limits the allowable bending motion in this plane commensurate with that allowed by a natural disc.

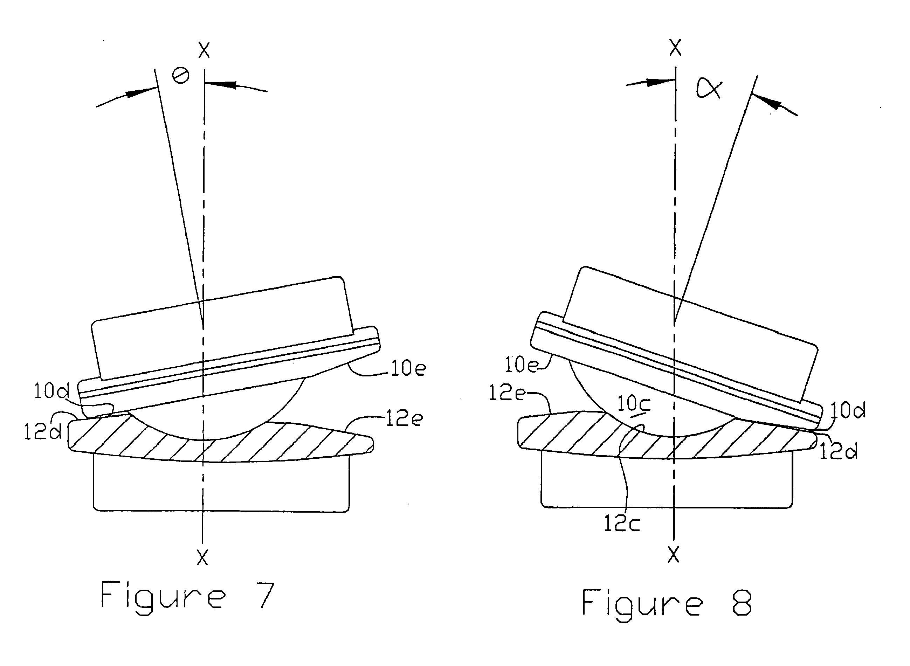

Figs. 7 and 8 illustrate the limited motion or bending allowed by the disc in the A/P plane. In the forward or anterior direction the rims contact each other when the components pivot relative to each other through an angle of ⊖ (Fig. 7) which is preferably the same as the angle β discussed above. The beveled portions 10e/12e allow the components to pivot relative to each other in the rearward or posterior direction though an angle of ∝ within the range of about 20° to 30°, i.e., at least 1.5θ. This extended rearward bending angle serves to inhibit the expulsion of the disc from between the separated vertebral bodies which might otherwise occur without the beveled portions.

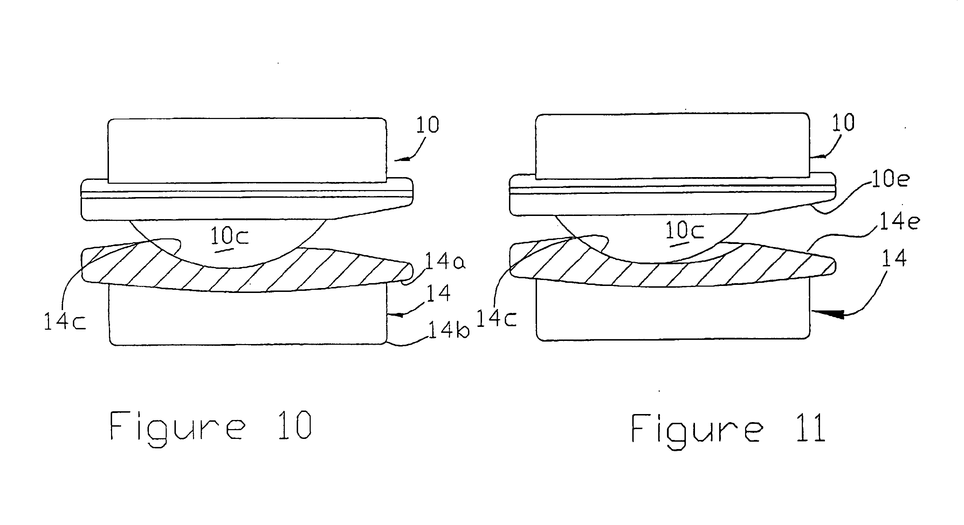

Figs. 9-11 illustrate an alternative embodiment of the disc in which the first component 10 remains the same, but the second or bottom component 14 is provided with a vertebral body engaging surface 14a on one side with keels 14b similar to that shown in Fig. 2 and a modified semispherical articulating recess 14c on the other side. A rim 14d similar to 12d encircles the recess 14c on the other side. The concave articulating surface or recess 14c has a radius in the medial plane which matches the radius of the spherical articulating surface 10c and a larger swept radius in the A/P plane as described in considerable detail in PCT Application No. US0616392. This allows for a generally line to line articulating contact with controlled rotation or bending in the A/P plane and controlled rotation/bending in the M/L plane.

There has thus been described a novel ball/socket type intervertebral prosthesis which allows rotational movement about the spinal axis, restricted bending of about +/-6° in each direction in the M/L plane and in the forward direction in the A/P plane. Optionally the prosthesis may accommodate additional bending of about 20° to 30° in the backward direction in the A/P plane.

Various modifications and perhaps improvements to the disclosed embodiments will undoubtedly occur to those skilled in the art without involving any departure from the spirit and scope of the invention as defined by the appended claims.

## Claims

1. A motion restoring intervertebral prosthesis for partially or completely replacing a defective natural spinal disc comprising:
a) a first component having a vertebral body engaging surface on one side for buttressing against a first vertebral body and an articulating surface on the other side in the form of an outwardly projecting, generally convex section joined to an encircling rim section; and
b) a second component having a vertebral engaging surface on one side for buttressing against an opposing vertebral body and a mating articulating surface on the other side in the form of an inwardly extending generally concave section joined to an encircling rim section, the components defining an imaginary longitudinal axis, the mating articulating surfaces being arranged to accommodate rotational motion about the longitudinal axis and pivoting action along the anterior-posterior and medial-lateral planes, the rim sections being arranged to contact each other to limit the angle through which the components can pivot relative to each other in at least the anterior-posterior plane in a forward direction and in the medial-lateral plane to about +/- 6° from the longitudinal axis.

2. The prosthesis of claim 1 wherein θ represents the pivot angle and θ is within the range of about +/- 4°.

3. The prosthesis of claim 2 wherein θ is within the range of about +/- 3°.

4. The prosthesis of claim 1 wherein the rim sections are not arranged to contact each other in the anterior-posterior plane in a rearward direction until the components have pivoted relative to each other through and angle within the range of about 20° to 30°.

5. The prosthesis of claim 4 wherein the first component's articulating surface is generally semispherical with a first radius and the second component's articulating surface has a radius in the M/L plane which matches the first radius and a larger swept radius in the A/P plane.

6. A motion restoring intervertebral prosthesis for partially or completely restoring a failed natural spinal disc comprising:
a) first and second components, each component having a vertebral body engaging surface on one side for buttressing against a respective separated vertebral body and an articulating surface on the other side;
b) the articulating surface of the first component in the form of an outwardly projecting, generally convex section joined to an encircling rim section;
c) the articulating surface of the second component in the form of an inwardly extending generally concave mating section joined to an encircling rim section;
d) the components defining an imaginary longitudinal axis with the mating articulating surfaces being arranged to accommodate rotational motion about the longitudinal axis and pivoting action along the anterior-posterior and medial-lateral planes;
e) the rim sections being arranged to contact each other to limit the angle through which the components can pivot relative to each other in at least the anterior-posterior plane in a forward direction and in both directions in the medial-lateral plane to an angle of +/- θ from the longitudinal axis; and
f) the rim sections being arranged to contact each other in the anterior-posterior plane in a rearward direction through an angle of ∝ which is at least 1.5θ.

7. The prosthesis of claim 6 wherein θ is within the range of about +/-3° to +/-6°.

8. A motion restoring prosthesis for replacing a partially or completely defective natural spinal disc comprising:
a) first and second components, the components, when assembled, defining an aligned imaginary longitudinal axis, and having a vertebral body engaging surfaces on one side thereof and mating articulating surfaces in the general form of a ball and socket on the other side;
b) the articulating surfaces being arranged to accommodate rotational motion about the longitudinal axis and limited pivoting action about the A/P and M/L planes; and
c) each component defining an annular rim section extending around the respective articulating surface, the rim sections being arranged to contact each other when the components have pivoted relative to each other along the A/P plane in a forward direction and the M/L plane in both directions through an angle θ of about +/-6°.

9. The prosthesis of claim 8 wherein the rims are arranged to contact each other when the components have pivoted relative to each other in a rearward direction in the A/P plane through angle ∝ of about 20° to 30°.

10. The prosthesis of claim 9 wherein θ =+/- 4°.
